# EUROPEAN PATENT SPECIFICATION

(11) **EP 0 439 442 B1**
(45) Date of publication and mention of the grant of the patent: **06.03.1996**
(21) Application number: 91870008.9
(22) Date of filing: 21.01.1991
(51) Int. Cl.: C12N 15/62, C12N 15/15

(54) **Factor x-laci hybrid protein**
Faktor X-LACI-Hybridprotein
Protéine hybride, facteur X-LACI

(30) Priority: 25.01.1990 US 470289
(43) Date of publication of application: 31.07.1991
(73) Proprietor: WASHINGTON UNIVERSITY, St. Louis, MO 63130 (US)
(72) Inventor: Girard, Thomas James, St Louis, MO 63117 (US); Broze, George John Jr., St Louis, MO 63131 (US)
(74) Representative: Ernst, Hubert

(56) References cited:
- EP-A- 0 318 451
- NATURE, vol. 338, 06 April 1989, London (GB); T.J. GIRARD et al., pp. 518-520
- BIOCHEMISTRY, vol. 29, no. 33, 21 August 1990, Easton, PA (US); G.J. BROZE et al., pp. 7539-7546
- SCIENCE, vol. 248, 15 June 1990, Lancaster, PA (US); T.J. GIRARD et al., pp. 1421-1424

## Description

### Background of the Invention

This invention relates to a novel hybrid protein and, more particularly, to a synthetic blood coagulation inhibitor having an amino acid sequence composed of two subsequences of human plasma Factor X and human Lipoprotein-Associated Coagulation Inhibitor (LACI), respectively.

The coagulation cascade that occurs in mammalian blood comprises two distinct systems - the so-called intrinsic and extrinsic systems. The latter system is activated by exposure of blood to tissue thromboplastin (Factor III), hereinafter referred to as tissue factor (TF). Tissue factor is a lipoprotein that arises in the plasma membrane of many cell types and in which the brain and lung are particularly rich. Upon coming into contact with TF, plasma Factor VII or its activated form, Factor VIIₐ, forms a calcium-dependent complex with TF and then proteolytically activates Factor X to Factor Xₐ , and Factor IX to Factor IXₐ, thereby triggering a cascade of events which leads eventually to the formation of thrombin and a fibrin clot.

Plasma contains a Lipoprotein-Associated Coagulation Inhibitor (LACI) which inhibits activated Factor X (Xa) directly and, in a Xa-dependent manner, inhibits VII(a)/TF activity, presumably by forming a quaternary Xa/LACI/VII(a)/TF complex. See, e.g., Broze et al., Blood 71, 335-343 (1988); Sanders et al., Ibid. 66, 204-212 (1985); and Hubbard et al., Thromb. Res. 44, 527-537 (1987).

Sequence analysis of complementary DNA clones has shown that LACI contains three tandemly repeated Kunitz-type serine protease inhibitory domains. See, e.g. Wun et al., J. Biol. Chem. 263, 6001-6004 (1988); Girard et al., Thromb. Res. 55, 37-50 (1988); Girard et al., Nature 338, 518-520 (1989); and European Patent Application No. 318,451, published May 31, 1989. Both the first and second Kunitz domains of LACI are necessary for inhibition of VII(a)/TF. The present inventors have proposed that in the putative Xa/LACI/VII(a)/TF inhibitory complex, LACI's first Kunitz domain is bound to the active site of VII(a)/TF while the second Kunitz domain is bound to Xa's active site as reported in Nature 338, 518-520 (1989).

Coagulation Factor X (Stuart factor) is a two chain molecule covalently linked by a disulfide bridge. Proteolytic release of a peptide from the heavy chain of zymogen Factor X through the action of VII(a)/TF (or Factor IXa with its cofactor VIIIa) produces the active enzyme Xa. The heavy chain of Xa contains the catalytic site whereas Xa's light chain contains an NH₂-terminal, γ-carboxyglutamic acid (gla)-containing domain, which is responsible for Ca⁺⁺ binding, followed by two growth factor-like domains which may in part mediate its interaction with specific coagulation cofactors. Chymotryptic treatment of bovine Xa cleaves the gla-containing domain from the NH₂-terminus of the molecule (residues 1-44), and although LACI binds to and inhibits gla-domainless Xa, Xa(-GD), LACI in the presence of Xa(-GD) does not inhibit VII(a)/TF activity.

### Brief Description of the Invention

In accordance with the present invention a novel single-chain hybrid protein is provided which is a potent blood coagulation inhibitor. This hybrid protein has been synthesized whereby it has an amino acid sequence composed of two subsequences of human plasma Factor X and human Lipoprotein-Associated Coagulation Inhibitor (LACI), respectively. In this hybrid, protein, Factor Xa's light chain is fused to LACI's first Kunitz domain.

In a preferred embodiment, a genetically engineered hybrid protein, designated X_{LC}LACI_{Kl}, consisting of the light chain of Factor X and LACI's first Kunitz domain, which inhibits VII(a)/TF activity directly, was prepared. In contrast to LACI's mode of action, inhibition of VII(a)/TF activity by X_{LC}LACI_{Kl} is not Xa dependent. X_{LC}LACI_{Kl} expressed by cells grown in the presence of warfarin, which inhibits γ-carboxylation of the hybrid protein, dramatically reduced inhibitory activity, thereby indicating the LACI_{Kl} portion of the hybrid protein alone was not sufficient for its inhibitory activity.

In a preferred embodiment to prepare X_{LC}LACI_{Kl} , conventional recombinant DNA procedures were used to design a hybrid gene encoding for Factor X's proleader sequence (which directs the γ-carboxylation of glutamic acids) and light chain fused to a sequence encoding LACI's first Kunitz domain.

Using a previous numbering system in which amino acid +1 was assigned to the first methionine after a stop codon in the 5′-noncoding region, the first Kunitz domain was originally designated as LACI(47-117). See European Patent Application No. 318,451, published May 31, 1989, and Wun et al., J. Biol. Chem. 263, 6001-6004 (1988). In a preferred numbering system in which amino acid +1 is assigned to the NH₂-terminus of the 276 amino acid LACI protein, the first Kunitz domain can be designated LACI(19-89). See Girard et al., Nature 338, 518-520 (1989). It will be appreciated, however, that variations in length and composition of the first Kunitz domain or the individual internal amino acids which do not adversely or detrimentally affect the biological activity of the hybrid protein as defined herein are included within the scope of the invention. Thus, in the illustrative embodiment, X_{LC}LACI_{Kl}, the first Kunitz domain in the hybrid protein is a subsequence of 79 amino acids which includes LACI(12-88) with an additional Met-His at the C-terminus.

In a like manner, the light chain sequence of Factor X in the hybrid protein which includes the proleader sequence (residues -40 to -1) and the light chain sequence (residues 1 to 131) for a total of 171 amino acids in the preferred embodiment, X_{LC}LACI_{Kl}, can also vary in length and composition or in the individual internal amino acids to the extent that it does not adversely or detrimentally affect the biological activity of the hybrid protein as defined herein.

The cDNA sequence of a hybrid gene encoding for the X_{LC}LACI_{Kl} hybrid protein is shown in the following 788 bp sequence which includes portions of the 5′- and 3′-noncoding regions. Nucleotides are numbered above the cDNA sequence. The corresponding 250 amino acids of the hybrid protein are shown below the cDNA sequence.

10 20 30 40 50 60
GGATCCGAATTCGGATCCACCATGGGGCGCCCACTGCACCTCGTCCTGCTCAGTGCCTCC
M G R P L H L V L L S A S

70 80 90 100 110 120
CTGGCTGGCCTCCTGCTGCTCGGGGAAAGTCTGTTCATCCGCAGGGAGCAGGCCAACAAC
L A G L L L L G E S L F I R R E Q A N N

130 140 150 160 170 180
ATCCTGGCGAGGGTCACGAGGGCCAATTCCTTTCTTGAAGAGATGAAGAAAGGACACCTC
I L A R V T R A N S F L E E M K K G H L

190 200 210 220 230 240
GAAAGAGAGTGCATGGAAGAGACCTGCTCATACGAAGAGGCCCGCGAGGTCTTTGAGGAC
E R E C M E E T C S Y E E A R E V F E D

250 260 270 280 290 300
AGCGACAAGACGAATGAATTCTGGAATAAATACAAAGATGGCGACCAGTGTGAGACCAGT
S D K T N E F W N K Y K D G D Q C E T S

310 320 330 340 350 360
CCTTGCCAGAACCAGGGCAAATGTAAAGACGGCCTCGGGGAATACACCTGCACCTGTTTA
P C Q N Q G K C K D G L G E Y T C T C L

370 380 390 400 410 420
GAAGGATTCGAAGGCAAAAACTGTGAATTATTCACACGGAAGCTCTGCAGCCTGGACAAC
E G F E G K N C E L F T R K L C S L D N

430 440 450 460 470 480
GGGGACTGTGACCAGTTCTGCCACGAGGAACAGAACTCTGTGGTGTGCTCCTGCGCCCGC
G D C D Q F C H E E Q N S V V C S C A R

490 500 510 520 530 540
GGGTACACCCTGGCTGACAACGGCAAGGCCTGCATTCCCACAGGGCCCTACCCCACAGAT
G Y T L A D N G K A C I P T G P Y P T D

550 560 570 580 590 600
ACGGAGTTGCCACCACTGAAACTTATGCATTCATTTTGTGCATTCAAGGCGGATGATGGC
T E L P P L K L M H S F C A F K A D D G

610 620 630 640 650 660
CCATGTAAAGCAATCATGAAAAGATTTTTCTTCAATATTTTCACTCGACAGTGCGAAGAA
P C K A I M K R F F F N I F T R Q C E E

670 680 690 700 710 720
TTTATATATGGGGGATGTGAAGGAAATCAGAATCGATTTGAAAGTCTGGAAGAGTGCAAA
F I Y G G C E G N Q N R F E S L E E C K

730 740 750 760 770 780
AAAATGTGTACAAGAGATAATGCAAACAGGATTATAAAGACAACAATGCATTGATGAAGC
K M C T R D N A N R I I K T T M H *

790 800 810 820 830 840
TTGGATCC

In the preferred embodiment illustrated herein, the hybrid gene was inserted into a bovine papilloma virus (BPV) expression vector and the gene was transfected into mouse C127 fibroblasts. Use of BPV in a process for producing recombinant DNA suitable for introduction and replication in eukaryotic cells is well known as can be seen, e.g., from U.S. Patent 4,419,946. It will be appreciated that the novel hybrid gene can be used for transfection of other prokaryotic and eukaryotic cells, e.g. E. coli cells or in Chinese Hamster Ovary (CHO) cells and the like mammalian cells, for expression of the hybrid protein.

An illustrative example of a conventional and suitable BPV expression vector is the plasmid pMON1123. This vector is based on the 100% viral genome ligated to the well-known pBR322 derivative of pML2 and utilizes the mouse metallothionein I promoter and the SV40 Late poly A addition site to regulate the expression of foreign genes. Its use for the expression of LACI is described by Girard et al., Thromb. Res. 55, 37-50 (1989).

### Detailed Description of the Invention

While the specification concludes with claims particularly pointing out and distinctly claiming the subject matter regarded as forming the present invention, it is believed that the invention will be better understood from the following detailed description of preferred embodiments of the invention taken in conjunction with the appended drawings, in which, briefly:

FIG. 1 is a schematic diagram of an illustrative hybrid protein of the invention, designated X_{LC}LACI_{Kl}. The layout of Factor X light chain structure is based on Leytus et al., Biochemistry 25, 5098-5102 (1986). The LACI-derived portion of the hybrid protein is shaded and is based on Girard et al., Nature 338, 518-520 (1989). The arrow indicates the probable cleavage site to generate the mature protein.

FIG. 2 shows Western blots which illustrate the expression of the recombinant hybrid protein. Relative molecular masses of standards are shown at the right.

FIG. 3 shows SDS-PAGE and silver staining of the purified X_{LC}LACI_{Kl} (250 ng). The reduced sample contained 1.25% 2-mercaptoethanol. Relative molecular masses of standards are shown at the right.

FIG. 4 is a graphical representation which shows inhibition of VII(a)/TF by purified LACI in the presence (●); or absence (○) of 0.1 µg/ml Factor X.

FIG. 5 is a graphical representation which shows inhibition of VII(a)/TF activity by barium sulfate eluted X_{LC}LACI_{Kl} in the presence (●); or absence (○) of 0.1 µg/ml factor X.

FIG. 6 is a graphical representation which shows inhibition of TF-induced coagulation by purified X_{LC}LACI_{Kl} (○); and LACI (●).

FIG. 7 shows a Western blot which illustrates the presence of X_{LC}LACI_{Kl} in the media of C10.1 cells cultured with vitamin K or warfarin.

FIG. 8 is a schematic of the proposed inhibitory complexes with LACI and with X_{LC}LACI_{Kl}. Indentations represent the active sites for VII(a) and Xa; and the protrusions represent LACIs' three Kunitz domains. In the quaternary Xa/LACI/VII(a)/TF complex, Xa is bound at its active site to LACI's second Kunitz domain and VII(a) is bound at its active site to the first Kunitz domain of LACI. Xa's gla domain interacts in a Ca⁺⁺ dependent fashion with an as yet to be identified site of the VIIa/TF complex. The X_{LC}LACI_{Kl} hybrid protein likely substitutes for the Xa/LACI complex.

Standard biochemical nomenclature is used herein in which the nucleotide bases are designated as adenine (A); thymine (T); guanine (G); and cytosine (C). Corresponding nucleotides are, for example, deoxyguanosine-5′-triphosphate (dGTP). As is conventional for convenience in the structural representation of a DNA nucleotide sequence, only one strand is shown in which A on one strand connotes T on its complement and G connotes C.

Amino acids are shown herein either by three letter or one letter abbreviations as follows:

| Abbreviated Designation | | Amino Acid |
|---|---|---|
| A | Ala | Alamine |
| C | Cys | Cysteine |
| D | Asp | Aspartic acid |
| E | Glu | Glutamic acid |
| F | Phe | Phenylalanine |
| G | Gly | Glycine |
| H | His | Histidine |
| I | Ile | Isoleucine |
| K | Lys | Lysine |
| L | Leu | Leucine |
| M | Met | Methionine |
| N | Asn | Asparagine |
| P | Pro | Proline |
| Q | Gln | Glutamine |
| R | Arg | Arginine |
| S | Ser | Serine |
| T | Thr | Threonine |
| V | Val | Valine |
| W | Trp | Tryptophan |
| Y | Tyr | Tyrosine |

Commonly available restriction endonucleases described herein have the following restriction sequences and (indicated by arrows) cleavage patterns:

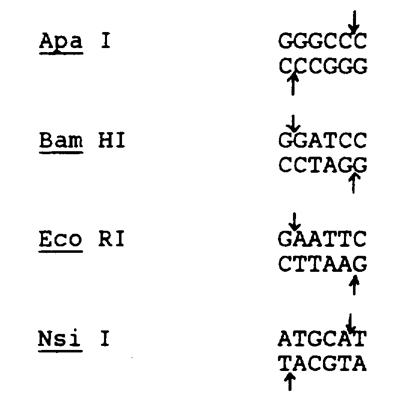

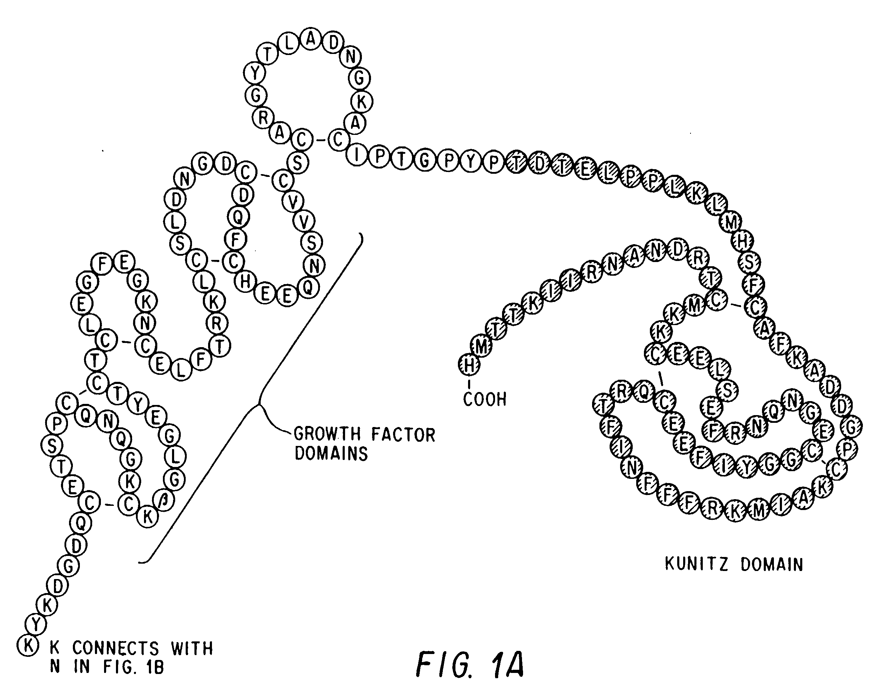

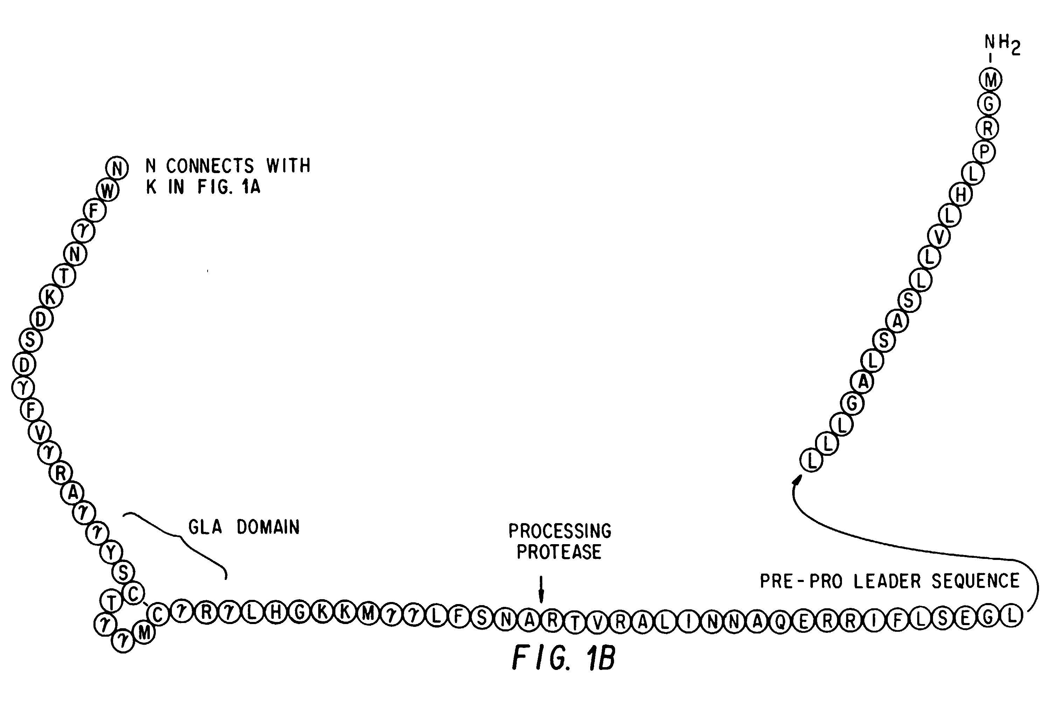

In the sequences shown in FIG. 1, γ refers to γ-carboxyglutamic acid, and β refers to β-hydroxyaspartic acid.

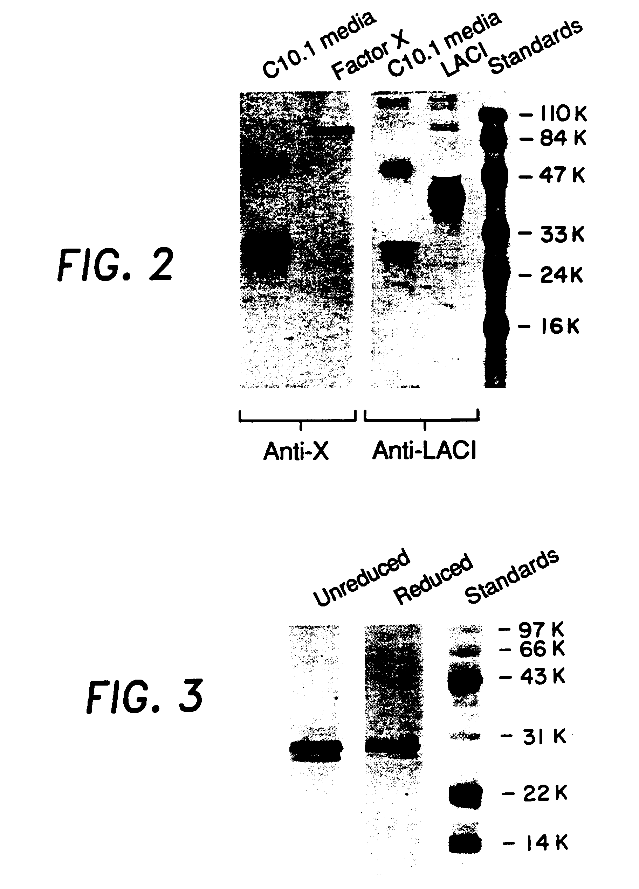

In a preferred embodiment of the invention, a hybrid gene encoding for Factor X's preproleader sequence (which directs the γ-carboxylation of glutamic acids) and light chain fused to a sequence encoding LACI's first Kunitz domain was designed (Fig. 1). Inserted into a bovine papilloma virus expression vector, this gene was transfected into mouse C127 fibroblasts. Expression of the hybrid protein molecules into the media by a transfected clone, designated C10.1, was detected using Western blot analysis. Both a monoclonal antibody to Xa's light chain and a polyclonal IgG fraction to the NH₂-terminal sequence of LACI resulted in similar staining patterns with the dominantly stained material migrating at 30,000 molecular weight (MW) and a less intensely stained band at 28,000 MW (Fig. 2). A diffuse band with MW 52,000 was also observed and although it could represent oligomeric hybrid complexes, the identification of this material remains unclear.

SDS-PAGE and silver staining showed that the purified hybrid protein consists of a dominant 30,000 MW band with minor bands of 31,000 and 28,000 MW. This staining pattern was also observed under reducing conditions, thereby indicating that the multiple bands are not simply due to alternative disulfide bonding (Fig. 3).

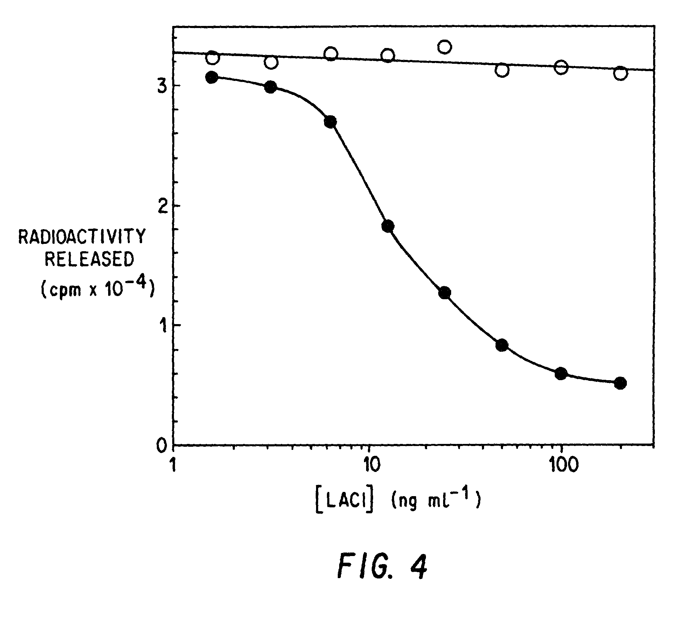

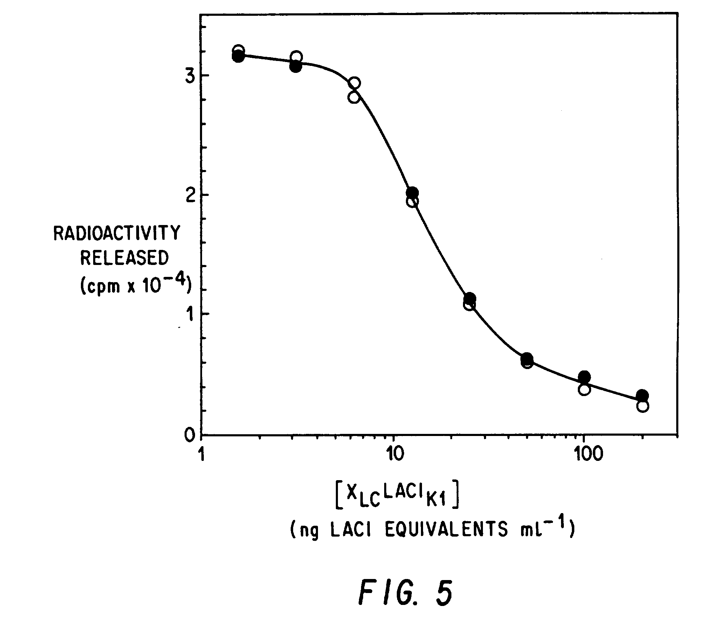

The ability of the expressed X_{LC}LACI_{Kl} molecules to inhibit VII(a)/TF activity was investigated. VII(a)/TF activity was determined by the release of the activation peptide from its substrate factor IX [Girard et al., Thromb. Res. 55, 37-50 (1988)]. Assaying extracted gel slices following SDS-PAGE of the C10.1 cells' conditioned media showed that functional inhibitory activity co-migrates with immuno-stained material having a MW of 30,000. No inhibitory activity was observed for the 28,000 or 52,000 MW materials. As previously demonstrated [Broze et al., Blood 71, 335-343 (1988)], Fig. 4 illustrates that the inhibition of VII(a)/TF activity by LACI is dependent upon the presence of Factor Xa. In contrast, inhibition of VII(a)/TF activity by the X_{LC}LACI_{Kl} Hybrid was not dependent on Xa (Fig. 5).

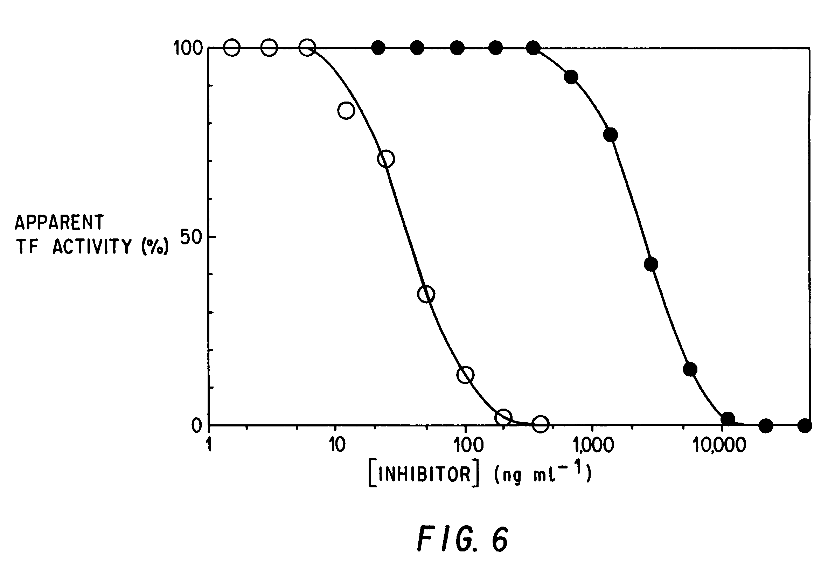

X_{LC}LACI_{Kl} was compared to LACI for the ability to inhibit TF-induced coagulation of normal plasma as measured in a modified prothrombin time assay. Fig. 6 shows that 50% apparent TF inhibition occurred with 35 ng/ml X_{LC}LACI_{Kl} while 2.5 µg/ml LACI was required for the equivalent effect. The greater relative inhibitory activity of X_{LC}LACI_{Kl} in this system is believed to reflect its ability to directly inhibit VIIa/TF while LACI requires the generation of Xa before its inhibition of VIIa/TF becomes manifest. Further, the inhibitory effect of LACI in the prothrombin time assay is at least partly due to its direct inhibition of Xa since at the same concentrations, LACI also prolongs surface contact activated coagulation of normal plasma, as measured by the activated partial thromboplastin time (aPTT).

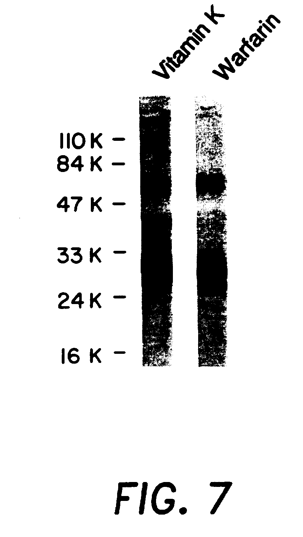

The γ-carboxylation of glutamic acid residues on Factor X's light chain requires a vitamin K dependent process which is inhibited by warfarin. Replacement of vitamin K with warfarin in the C10.1 culture media results in an ∼ 80 fold reduction in VII(a)/TF inhibitory activity (Table 1), while reducing the quantity of X_{LC}LACI_{Kl} in the media only ∼ two fold (Fig. 7). Barium sulfate, which selectively adsorbs gla-containing molecules [Kisiel & Davies, Biochemistry 14, 4928-4934 (1975)], binds the VII(a)/TF inhibitory activity expressed by C10.1 cells grown in the presence of vitamin K, indicating that the functional X_{LC}LACI_{Kl} is γ-carboxylated (Table 1). Thus the γ-carboxylation of X_{LC}LACI_{Kl} appears essential for its VII(a)/TF inhibitory activity, and it is concluded by the inventors that the LACI_{Kl} portion of the hybrid protein alone is not sufficient for the observed inhibitory activity. In addition, neither Factor X, Xa nor inactivated Xa (Xa treated with dansyl-L-glutamyl-L-glycyl-L-arginine chloromethyl ketone) possessed VII(a)/TF inhibitory activity though each contains the X light chain found in the X_{LC}LACI_{Kl} hybrid. These results indicate both the X light chain and LACI_{Kl} portions of the hybrid protein are necessary for VII(a)/TF inhibitory activity.

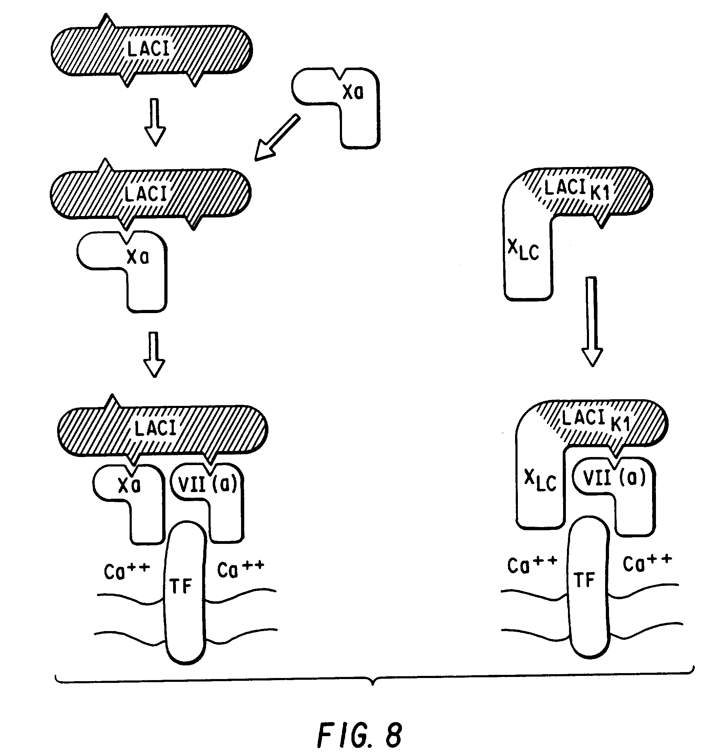

A schematic illustrating the formation of the Xa/LACI/VII(a)/TF and X_{LC}LACI_{Kl}/VII(a)/TF inhibitory complexes is shown in Fig. 8. It is believed by the inventors that the X_{LC}LACI_{Kl} mimics the Xa/LACI complex in binding to and inhibiting VIIa/TF. LACI's inhibition of VII(a)/TF activity occurs by a novel feedback inhibition mechanism requiring the generation of Xa, a product of VII(a)/TF catalytic activity. In contrast, the X_{LC}LACI_{Kl} hybrid protein inhibits VII(a)/TF activity directly.

In order to illustrate the invention in greater detail the following preparative laboratory work was carried out to generate the results set forth herein, including the appended drawings. The methods described are subdivided for relation to the results shown in FIGS. 1 to 7 and Table 1. It will be appreciated, however, that the invention is not limited to these specific examples.

### EXAMPLES

### FIG. 1

Site-directed mutagenesis was performed on a modified LACI cDNA insert [Girard et al., Nature 338, 518-520 (1989)] to create a Nsi I site between LACI's first and second Kunitz domains' coding sequences. A naturally occurring Nsi I site exists upstream from the first Kunitz domain's coding sequence. Following Nsi I digestion, the region encoding LACI's first Kunitz domain was isolated and ligated into the NsiI site of pGEMX_{LC} to create pGEMX_{LC}LACI_{Kl}. pGEMX_{LC} was derived from a commercially available plasmid, pGEM (Promega, Madison, WI) containing a cDNA insert encoding for human Factor X with the naturally occurring Eco R1 site eliminated by a modification which does not alter the encoded amino acid sequence (from A. Strauss, Washington University, St. Louis, MO). pGEMX_{LC} contains a modified partial cDNA encoding for the light chain of Factor X lined via an Apa I site to complementary synthetic oligomers. The synthetic oligomers encode for additional Factor X light chain sequence, followed by LACI NH₂-terminal sequence which precedes the first Kunitz domain and includes the naturally occurring Nsi I site, followed by two stop codons and convenient restriction site palindromes. The pGEMX_{LC}LACI_{Kl} insert was excised with Bam HI and ligated into the bovine papilloma virus expression vector pMON1123 and the resulting plasmid, pMONX_{LC}LACIₗ, was used with pSV2neo to co-transfect C127 mouse fibroblasts [Girard et al., Thromb. Res. 55, 37-50 (1988)].

### FIG. 2

For the Western blots, five fold concentrated serum-free conditioned media (25 µl) from a transfected cloned cell line, designated C10.1, and 40 ng purified LACI or 60 ng Factor X were run on a 15% polyacrylamide gel, transferred to nitrocellulose, probed with anti-LACI_{(peptide)} IgG or anti-X monoclonal antibody and colorimetrically developed by conventional procedure as described by Girard et al., Thromb. Res. 55, 37-50 (1988).

### FIG. 3

X_{LC}LACI_{Kl} was purified from C10.1 serum free conditioned media by barium sulfate adsorption and elution [Kisiel & Davies, Biochemistry 14, 4928-4934 (1975)], followed by anhydrotrypsin affigel affinity chromatography [Ishii et al., Meth. Enzymol. 91, 378-383 (1983)], and Mono Q anion exchange chromatography [copending application Ser. No. 07/77,366, filed July 23, 1987 and Broze et al., Blood 71, 335-343 (1988)].

### FIGS. 4 and 5

The purification of LACI was carried out by conventional procedure as described by Broze et al., Thromb. Res. 48, 253-259 (1988). X_{LC}LACI_{Kl} was partially purified from 200 ml of C10.1 serum-free conditioned media by barium sulfate adsorption [Kisiel & Davies, supra.] and eluted with 0.2 M sodium citrate. The preparation was then concentrated and dialyzed into TS buffer (100 mM NaCl, 50 mM tris-HCl, pH 7.4); final volume = 1 ml. VII(a)/TF inhibition was assayed using a previously described ³H-IX activation peptide release assay [Girard et al., Thromb. Res. 55, 37-50 (1988)] except that the Factor VII in the assay was replaced with VIIa since in some assays no X (or Xa) is present to activate the VII to VIIa. Heparin (2 units/ml) was also present in the assay system. In this assay the activation of IX by VII(a)/TF is measured as release of TCA soluble tritiated activation peptide from radiolabeled IX and VII(a)/TF inhibitory activity results in decreased TCA soluble cpm. [TCA = trichloroacetic acid]. Nanograms LACI equivalents were determined from a standard LACI concentration curve.

### FIG. 6

TF-induced coagulation was measured by a modified prothrombin time assay in a fibrometer (BBL, Cockeysville, MD) by incubating 60 µl rabbit brain cephalin, 60 µl crude EDTA-washed TF [Broze & Majerus, J. Biol. Chem. 255, 1242-1247 (1980)], 10 µl sample and 60 µl normal human plasma (George King, Overland Park, KS) at 37°C. After 30 seconds, 60 µl CaCl₂ (25 mM) was added and the time to clot formation determined. In the absence of the inhibitors the clotting time of the assay was 29 seconds. A standard curve plotting TF concentration versus clotting time (log-log plot) was used to determine apparent TF activity at various inhibitor concentrations in the assay.

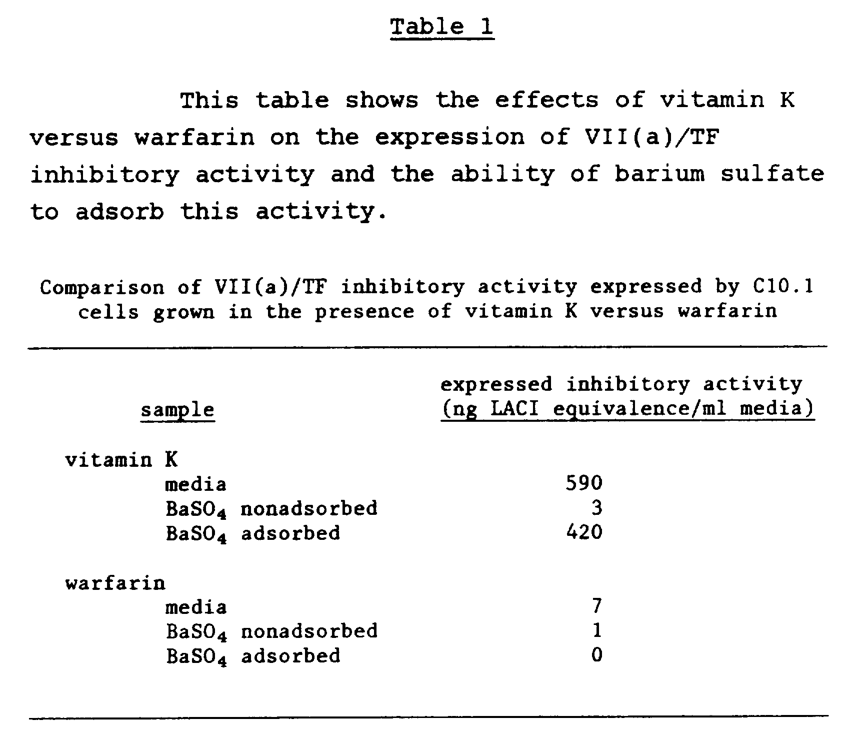

Upon reaching confluence, C10.1 cells expressing X_{LC}LACI_{Kl} were transferred to serum-free conditioned media containing vitamin K (1 µg/ml) or warfarin (20 µg/ml). The culture medias were replaced on days 0, 1 and 2 and harvested on day 5. Ten ml of each media were barium sulfate adsorbed as described under Fig. 3, above, and adsorbed proteins were eluted with 0.2 M sodium citrate. The barium sulfate adsorbed and eluted materials, barium sulfate nonadsorbed materials and 10 ml of additional conditioned medias were each dialyzed into TS buffer and concentrated; final volumes = 1 ml each. Samples were diluted appropriately in TBSA buffer and assayed for inhibition of VII(a)/TF activity. Nanograms LACI equivalents were determined from a standard LACI concentration curve.

### FIGURE 7

For the Western blot, 20 µl of each concentrated media was electrophoretically fractionated by SDS-PAGE, transferred to nitrocellulose, probed with an anti-X-monoclonal antibody followed by an anti-mouse IgG-alkaline phosphatase conjugate and developed colorimetrically.

Various other examples will be apparent to the person skilled in the art after reading the present disclosure without departing from the spirit and scope of the invention. It is intended that all such other examples be included within the scope of the appended claims.

## Claims

1. A single-chain hybrid protein having an amino acid sequence composed of two subsequences corresponding to the light chain of Factor X and LACI's first Kunitz domain.

2. A single-chain hybrid protein having an amino acid sequence as shown in FIG. 1 (Fig. 1A + Fig. 1B) of the drawings.

3. The single-chain hybrid protein of either Claim 1 or Claim 2 for use as a medicament.

4. The single-chain hybrid protein of either Claim 1 or Claim 2 for use as a blood coagulation inhibitor.

5. The single-chain hybrid protein of either Claim 1 or Claim 2 for use as a blood coagulation inhibitor in a mammal.

6. Use of a single-chain hybrid protein according to either Claim 1 or Claim 2 for the manufacture of a medicament for use as a blood coagulation inhibitor in a mammal.

7. A DNA sequence comprising a sequence encoding the hybrid protein of Claim 1.

8. A DNA sequence comprising a sequence encoding the hybrid protein of Claim 2.

## Patentansprüche

1. Einkettiges Hybridprotein mit einer Aminosäuresequenz, die aus zwei Untersequenzen zusammengesetzt ist, entsprechend der leichten Kette von Faktor X und der ersten Kunitz-Domäne von LACI.

2. Einkettiges Hybridprotein mit einer Aminosäuresequenz, wie in Fig. 1 (Fig. 1A + Fig. 1B) der Zeichnungen gezeigt.

3. Einkettiges Hybridprotein nach Anspruch 1 oder Anspruch 2 zur Verwendung als Medikament.

4. Einkettiges Hybridprotein nach Anspruch 1 oder Anspruch 2 zur Verwendung als Blutkoagulationsinhibitor.

5. Einkettiges Hybridprotein nach Anspruch 1 oder Anspruch 2 zur Verwendung als Blutkoagulationsinhibitor bei einem Säuger.

6. Verwendung eines einkettigen Hybridproteins nach Anspruch 1 oder Anspruch 2 zur Erzeugung eines Medikaments zur Verwendung als Blutkoagulationsinhibitor bei einem Säuger.

7. DNA-Sequenz, welche eine für das Hybridprotein des Anspruchs 1 codierende Sequenz aufweist.

8. DNA-Sequenz, welche eine für das Hybridprotein des Anspruchs 2 codierende Sequenz aufweist.

## Revendications

1. Protéine hybride à chaîne unique, dont la séquence d'acides aminés se compose de deux sous-séquences correspondant à la chaîne légère du facteur X et au premier domaine de Kunitz du LACI.

2. Protéine hybride à chaîne unique, dont la séquence d'acides aminés est représentée sur la figure 1 (figure 1A + figure 1B) des dessins.

3. Protéine hybride à chaîne unique, conforme à l'une ou l'autre des revendications 1 et 2, destinée à être utilisée comme médicament.

4. Protéine hybride à chaîne unique, conforme à l'une ou l'autre des revendications 1 et 2, destinée à être utilisée comme inhibiteur de la coagulation du sang.

5. Protéine hybride à chaîne unique, conforme à l'une ou l'autre des revendications 1 et 2, destinée à être utilisée chez un mammifère comme inhibiteur de la coagulation du sang.

6. Emploi d'une protéine hybride à chaîne unique, conforme à l'une ou l'autre des revendications 1 et 2, pour la préparation d'un médicament destiné à être utilisé chez un mammifère comme inhibiteur de la coagulation du sang.

7. Séquence d'ADN comportant une séquence codant une protéine hybride conforme à la revendication 1.

8. Séquence d'ADN comportant une séquence codant une protéine hybride conforme à la revendication 2.
